(19) Europäisches Patentamt
European Patent Office
Office européen des brevets

(11) Publication number: 0 127 203 B1

(12) # EUROPEAN PATENT SPECIFICATION

(45) Date of publication of patent specification: 28.06.89

(51) Int. Cl.$^4$: C 07 D 307/88, B 41 M 5/12

(21) Application number: 84200378.2

(22) Date of filing: 08.04.82

(60) Publication number of the earlier application in accordance with Art. 76 EPC: 0 062 544

(54) **New phthalide derivatives, process for preparing the same and recording system utilizing the same as colourless chromogenic material.**

(30) Priority: 08.04.81 JP 53678/81
13.03.82 JP 39965/82

(43) Date of publication of application:
05.12.84 Bulletin 84/49

(45) Publication of the grant of the patent:
28.06.89 Bulletin 89/26

(84) Designated Contracting States:
CH DE FR GB LI

(56) References cited:
US-A-4 020 056
US-A-4 022 771
US-A-4 119 776

(73) Proprietor: KANZAKI PAPER MANUFACTURING CO., LTD
9-8 Yonchome Ginza Chuo-ku
Tokyo 104 (JP)

(72) Inventor: Kondo, Mitsuru
c/o Kanzaki Paper Manufacturing Co., Ltd.
1-11 Motomachi Jyokoji Amagaski Hyogo (JP)
Inventor: Okimoto, Tomoyuki
c/o Kanzaki Paper Manufacturing Co., Ltd.
1-11 Motomachi Jyokoji Amagaski Hyogo (JP)
Inventor: Kanda, Nobuo
c/o Kanzaki Paper Manufacturing Co., Ltd.
1-11 Motomachi Jyokoji Amagaski Hyogo (JP)

(74) Representative: Seaborn, George Stephen et al
c/o Edward Evans & Co. Chancery House
53-64 Chancery Lane
London WC2A 1SD (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 127 203 B1

**Description**

Field of the Invention

The present application is divided out of European patent application no. 82301885.8 (publication no. 0 062 544).

This invention relates to phthalide derivatives as new compounds useful as colourless chromogenic materials, a process for preparing the same, and a recording system utilizing the same.

Background of the Invention

There are known various kinds of record systems utilizing the colour-forming reaction between a colourless chromogenic material and an electron accepting acidic reactant material by means of mechanical, heat, electric or light energy. Among them there are included a pressure sensitive recording sheet, a heat sensitive record sheet, an eletrothermal recording sheet, an ultrasonic record sheet, an electron beam recording sheet, an electrostatic record sheet and a photosensitive recording sheet. The colourless chromogenic materials of these kinds also find their usefulness in typewriter ribbons, ball-point pen ink, crayon and stamp ink.

One of the most typical colourless chromogenic materials is crystal violet lactone. This dye material reacts with an electron accepting acidic reactant material upon contact to develop a clear colour of bluish violet but the developed colour has a poor light resistance so that the recording images (colour images) soon disappear in a short time when subjected to radiation of ultraviolet rays of daylight. Another disadvantage of this type of dye is in the fact that the recorded images obtained with this material show no absorption for the infrared range of 700—900 nm and accordingly this type of dye material cannot be used for a reading machine utilizing an optical reading system responsive to infrared absorption.

US—A—4,022,771 discloses phthalide compounds having structure similar to that of the compounds according to the present invention. However, as compared with the compounds of the present invention the phthalide compounds in the US specification have amino group instead of the substituent or substituents $R_7$ defined below. The phthalide compounds according to the present invention are good in light resistance due to the substituent or substituents $R_7$.

US—A—4,020,056 discloses phthalide compounds having two vinyl groups in a molecule. Such phthalide compounds are different from the phthalide compounds according to the present invention in both structure and properties.

US—A—4,119,776 discloses phthalide compounds having a similar structure to that of the compounds according to the present invention except that the compounds of the US specification have a morpholino group instead of the substituent or substituents $R_7$.

The invention provides:

novel colourless chromogenic materials for use in recording systems in which the colour images when developed therefrom have a good light resistance, especially a good ultraviolet ray resistance and for infrared rays;

a process for preparing the aforementioned phthalide derivatives; and

an improved recording system in which a new phthalide derivative is used as a colourless chromogenic material and the colour images when developed therefrom have a good light resistance and show a good absorption for infrared rays.

The novel phthalide derivatives according to the invention have the general formula (I)

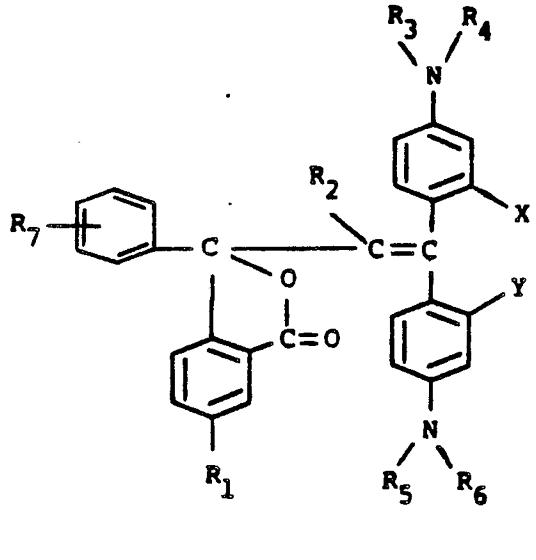

I

wherein

$R_1$ is hydrogen, $C_{1-4}$ alkoxy, nitro, pyrrolidino or a substituted amino group of the formula

$-N(CH_3)_2$, $-N(C_2H_5)_2$, 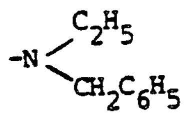 or 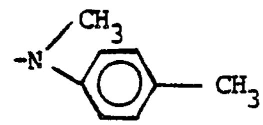 ;

$R_2$ is hydrogen or methyl;

each of $R_3$, $R_4$, $R_5$ and $R_6$ is $C_{1-4}$ alkyl, benzyl, halogen-substituted benzyl, phenyl, or $C_{1-2}$ alkyl or $C_{1-2}$ alkoxy-substituted phenyl, or $R_3$ and $R_4$ together with the adjacent nitrogen atom and/or $R_5$ and $R_6$ together with the adjacent nitrogen atom form pyrrolino, piperidino, hexamethyleneimino or morpholino;

$R_7$ is one or two substituents selected from $C_{1-4}$ alkyl, $C_{1-4}$ alkoxy and pyrrolidino;

each of X and Y is hydrogen, $CH_3$ or $OCH_3$.

The phthalide derivatives having the above general formula can be used as colourless chromogenic materials for use in various recording systems including a pressure sensitive recording system and a heat sensitive recording system. The compounds according to the invention can produce a colour of blue to green upon contact with an electron-accepting acidic reactant material. The colour images produced have a good light resistance and can maintain their clear colour tone initially produced for a long time. The colour images produced also show a good absorption for infrared rays with the range of 700—900 nm so that they can be detected for reading in an infrared ray responsive optical reading machine.

In a preferred aspect of the invention the novel phthalide derivatives are represented by the general formula (I) where

$R_1$ is a substituted amino group of the general formula

$-N(CH_3)_2$, $-N(C_2H_5)_2$, 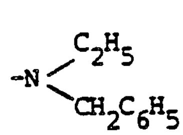 or, 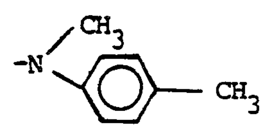 ;

and

$R_3$ and $R_5$ are the same, $R_4$ and $R_6$ are the same and X and Y are the same.

In another aspect of the invention the novel phthalide derivatives are represented by the general formula (II)

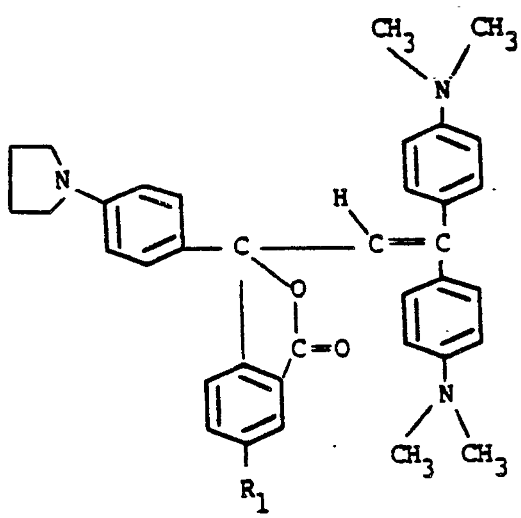

II

wherein $R_1$ is hydrogen, pyrrolidino, ethoxy or nitro compound of formula (III)

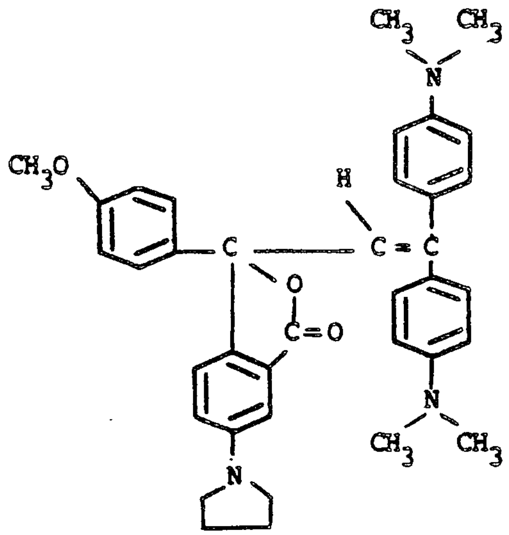

III

is specifically mentioned.

The compounds represented by the formula (I) may preferably be prepared by making benzophenone derivatives represented by the general formula (IV) react with ethylene derivatives represented by the general formula (V) with use of dehydration condensation agents at a temperature within the range of 30 to 150°C and for about 30 minutes to several hours:

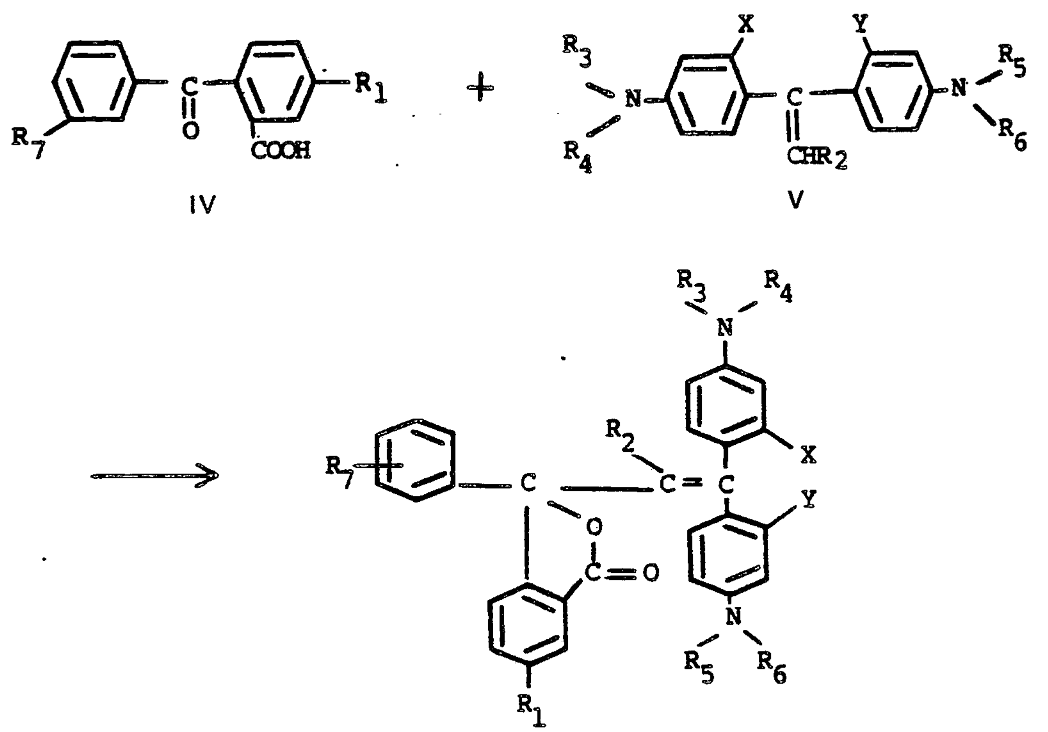

IV

V

I

wherein $R_1$, $R_2$, $R_3$, $R_4$, $R_5$, $R_6$, $R_7$, X and Y are as defined above.

The benzophenone derivatives represented by the general formula (IV) may preferably be prepared by dissolving 3-phenylphthalide derivatives represented by the general formula (VI) and then oxidizing them with use of nitrobenzene derivatives, or, by causing condensation between benzene derivatives represented by the general formula (VII) and phthalic anhydride derivatives represented by the general formula (VIII) in the presence of Friedel-Crafts type catalysts:

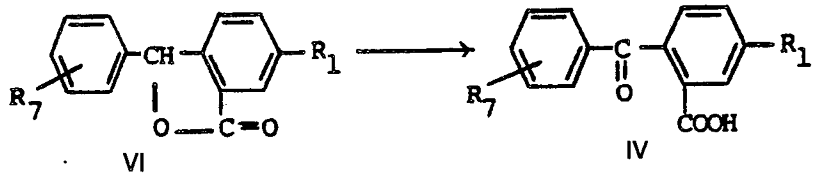

VI IV

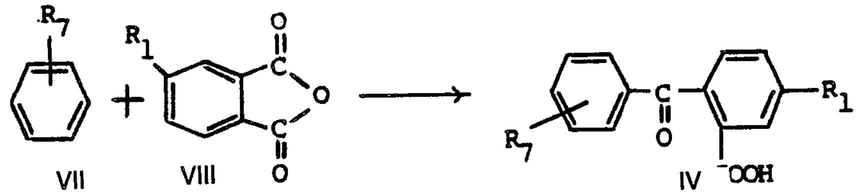

VII VIII IV

wherein $R_1$ and $R_7$ are the same as described above.

As typical benzophenone derivatives represented by the above-mentioned general formula (IV) which are used in this invention, the following compounds may be examplified: 2 - p - methylbenzoylbenzoic acid, 2 - p - methoxybenzoylbenzoic acid, 2 - p - pyrrolidinobenzoylbenzoic acid, 2 - o (or m or p) methylbenzoyl - 5 - dimethylaminobenzoic acid, 2 - o (or m or p) methoxybenzoyl - 5 - dimethylaminobenzoic acid, 2 - p - pyrrolidinobenzoyl - 5 - dimethylaminobenzoic acid, 2 - [2,4(or 3,4) - dimethyl]benzoyl - 5 - dimethylaminobenzoic acid, 2 - [2,4(or 3,4) - dimethoxy]benzoyl - 5 - dimethylaminobenzoic acid, 2 - (2 - methyl - 4 - methoxy)benzoyl - 5 - dimethylaminobenzoic acid, 2 - (2 - methoxy - 4 - methyl)benzoyl - 5 - dimethylaminobenzoic acid, 2 - p - methoxybenzoyl - 5 - diethylaminobenzoic acid, 2 - p - methoxybenzoyl - 5 - (N - ethyl - N benzyl)aminobenzoic acid, 2 - p - methoxybenzoyl - 5 - (N - methyl - N - p - tolyl)-aminobenzoic acid, 2 - p - methoxy - benzoyl - 5 - pyrrolidinobenzoic acid, 2 - p - pyrrolidinobenzoyl - 5 - ethoxybenzoic acid, 2 - p - pyrrolidinobenzoyl - 5 - pyrrolidinobenzoic acid, 2 - p - pyrrolidinobenzoyl - 5 - nitrobenzoic acid and the like.

Among the typical of ethylene derivatives represented by the above described general formula (V) which are used in this invention there may be included the following compounds: 1,1 - bis(p - dimethylaminophenyl)ethylene, 1,1 - bis(p - diethylaminophenyl)ethylene, 1,1 - bis - (p - - dimethylaminophenyl) - 1 - propene, 1,1 - bis(p - diethylaminophenyl) - 1 - propene, 1,1 - bis(4 - dimethylamino - 2 - methylphenyl)ethylene, 1,1 - bis(4 - diethylamino - 2 - methylphenyl)ethylene, 1,1 - bis(4 - dimethylamino - 2 - methoxyphenyl)ethylene, 1,1 - bis(4 - diethylamino - 2 - methoxyphenyl)ethylene, 1,1 - bis-[p - (N - methyl - N - benzyl)aminophenyl]ethylene, 1,1 - bis[p - (N - ethyl - N - benzyl)aminophenyl]-ethylene, 1,1 - bis[p - (N - ethyl - N - p - chlorobenzyl)aminophenyl]ethylene, 1,1 - bis[p - (N - methyl - N - phenyl)aminophenyl ethylene, 1,1 - bis[p - (N - methyl - N - p - tolyl)aminophenyl]ethylene, 1,1 - bis[p - (N - methyl - N - p - methoxyphenyl)aminophenyl]ethylene, 1,1 - bis[p - (N - methyl - N - p - methoxyphenyl)aminophenyl]ethylene, 1,1 - bis(4 - dimethylamino - 2 - methylphenyl) - 1 - propene, 1,1 - bis(4 - diethylamino - 2 - methylphenyl) - 1 - propene, 1,1 - bis(4 - dimethylamino - 2 - methoxyphenyl) - 1 - propene, 1,1 - bis(4 - diethylamino - 2 - methoxyphenyl) - 1 - propene, 1 - (4 - dimethylaminophenyl) - 1 - (4 - diethylaminophenyl)ethylene, 1 - (4 - dimethylaminophenyl) - 1 - (4 - diethylamino - 2 - methylphenyl)ethylene, 1 - (4 - dimethylaminophenyl) - 1 - (4 - diethylamino - 2 - methoxyphenyl)ethylene, 1 - (4 - dimethylaminophenyl) - 1 - (4 - N - benzyl - N - ethylaminophenyl)ethylene, 1 - (4 - dimethylaminophenyl) - 1 - [4 - [N - p - tolyl - N - methyl)aminophenyl]ethylene, 1,1 - bis(4 - pyrrolidinophenyl)ethylene, 1,1 - bis(4 - piperidinophenyl)ethylene, 1,1 - bis(4 - hexamethyleneiminophenyl)ethylene, 1,1 - bis(2 - methyl - 4 - pyrrolidinophenyl)ethylene, 1,1 - bis(2 - methyl - 4 - piperidinophenyl)ethylene, 1,1 - bis(2 - methyl - 4 - morpholinophenyl)ethylene, 1,1 - bis(2 - methyl - 4 - hexamethyleneiminophenyl)ethylene, 1,1 - bis(2 - methoxy - 4 - pyrrolidinophenyl)ethylene, 1,1 - bis(2 - methoxy - 4 - piperidinophenyl)ethylene, 1,1 - bis(2 - methoxy - 4 - hexamethyleneiminophenyl)-ethylene, 1 - (4 - pyrrolidinophenyl) - 1 - (4 - dimethylaminophenyl)ethylene, 1 - (4 - piperidinophenyl) - 1 - (4 - dimethylaminophenyl)ethylene, 1 - (4 - hexamethyleneiminophenyl) - 1 - (4 - dimethylaminophenyl)ethylene, 1 - (4 - pyrrolidinophenyl) - 1 - (4 - N - ethyl - N - benzylaminophenyl)ethylene, 1 - (4 - piperidinophenyl) - 1 - (4 - N - ethyl - N - benzylaminophenyl)ethylene, 1 - (4 - hexamethyleneiminophenyl) - 1 - (4 - N - ethyl - N - benzylaminophenyl)ethylene, 1 - (4 - pyrrolidinophenyl) - 1 - (4 - N - methyl - N - p - tolylaminophenyl)ethylene, 1 - (4 - piperidinophenyl) - 1 - (4 - N - methyl - N - p - tolylaminophenyl)ethylene, 1 - (4 - hexamethyleneiminophenyl) - 1 - (4 - N - methyl - N - p - tolylaminophenyl)ethylene, 1 - (4 - pyrrolidinophenyl) - 1 - (2 - methyl - 4 - diethylaminophenyl)ethylene, 1 - (4 - pyrrolidinophenyl) - 1 - (2 - methoxy - 4 - diethylaminophenyl)ethylene, 1,1 - bis(4 - pyrrolidinophenyl) - 1 - propene, 1,1 - bis(4 - piperidinophenyl) - 1 - propene and 1,1 - bis(4 - hexamethyleneiminophenyl) - 1 - propene.

The dehydration condensation agents which are used for carrying out the reaction between benzophene derivatives represented by the general formula (IV) and ethylene derivatives represented by the general formula (V) may preferably have a function as a solvent. Among typically useful dehydration condensation agents for this purpose there are included lower fatty acid anhyrides, e.g., acetic anhydride

and propionic anhydride, and inorganic acids, e.g., phosphorus oxychloride, phosphorus trichloride, sulfuric acid and polyphosphoric acid. Various Friedel-Crafts type catalysts may also be used as dehydration condensation agents. These dehydration condensation agents may be used either alone or in combination.

The phthalide derivates thus obtained according to the invention are substantially colourless chromogenic compounds which can develop a colour upon contact with electron accepting acidic materials (hereinafter referred to as "acceptors"). They are especially advantageous in that they have good light resistance and show a good absorption for infrared rays. Accordingly, they find their usefulness in use for the production of various kinds of record materials.

US—A—4,022,771 discloses similar compounds to the phthalide derivatives according to the invention as a chromogenic material useful to produce heat- and pressure-sensitive record materials. However, the phthalide derivatives represented by the formula (I) according to the invention are very superior in the colour developing rate upon contact with electron accepting materials to the known compounds. Further, the developed colour images in the heat-sensitive record materials with the use of phthalide derivatives according to the invention together with an acceptor such as 4,4'-isopropylidenediphenol, benzyl-4-hydroxybenzoate have a superior light resistance, and the developed colour is generally preferably colour for recorded images, such as dark blue, and blackish blue.

The above mentioned phthalide derivatives according to the invention may be used either solely or in combination or, when desired, together with any of the following basic dye compounds: triarylmethane-lactone compounds such as 3,3 - bis(p - dimethylaminophenyl) - 6 - dimethylaminophthalide, 3 - (p - dibenzylaminophenyl) - 3 - (1,2 - dimethylindole - 3 - yl) - 7 - azaphthalide, 3 - (4 - diethylamino - 2 - ethoxyphenyl)- 3 - (1 - ethyl - 2 - methylindole - 3 - yl) - 7 - azaphthalide and 3,3 - bis(1 - ethyl - 2 - methylindole - 3 - yl)phthalide; fluran compounds such as 3 - diethylamino - 6 - methylfluoran, 3 - diethylamino - 6 - methyl - 7 - chlorofluoran, 3 - (N - ethyl - N - p - tolylamino) - 7 - methylfluoran, 3 - diethylamino - 6 - methyl - 7 - anilinofluoran, 3 - (N - cyclohexyl - N - methylamino) - 6 - methyl - 7 - anilinofluoran, 3 - (N - ethyl - N - p - tolylamino) - 6 - methyl - 7 - anilinofluoran, and 3 - diethylamino - 7 - o - chloroanilinofluoran; spiropyran compounds such as di - β - naphthospiropyran and 3-methyl - di - β - naphthospiropyran; diphenylmethane compounds such as 4,4' - bis - dimethylamino-benzhydrylbenzyl ether, and 4,4' - bis - dimethylaminophenyl - p - toluenesulfinic acid ester; azine compounds such as 3,7 - bis(dimethylamino) - 10 - benzoyl-phenothiazine and 3,7 - bis(diethylamino) - 10 - benzoylphenoxazine; triarylmethane compounds such as N - butyl - 3 - [bis - {4 - (N - methyl - anilino)phenyl}methyl]carbazole.

The acceptors used are selected according to the kinds of recording materials. The materials which are preferably used as acceptors for pressure sensitive record materials, heat sensitive record materials, electrothermal record material, ultrasonic record materials, electrostatic record materials, typewriter ribbons, ball-point pen ink and crayon are those which function as Brønsted or Lewis acid. Among them there are included: inorganic acceptors such as acid clay, activated clay, attapulgite, bentonite, colloidal silica, aluminium silicate, magnesium silicate, zinc silicate, tin silicate, calcined kaolin and talc; organic acceptors such as aliphatic carboxylic acids, e.g. oxalic acid, maleic acid, tartaric acid, citric acid, succinic acid and stearic acid, aromatic carboxylic acids, e.g., benzoic acid, p-tert-butylbenzoic acid, phthalic acid, gallic acid, salicylic acid, 3-isopropylsalicylic acid, 3-phenylsalicyclic acid, 3-cyclohexyl-salicyclic acid, 3,5-di-tert-butylsalicyclic acid, 3-methyl-5-benzylsalicyclic acid, 3-phenyl-5-(α,α,dimethylbenzyl)-salicyclic acid, 3,5-di-(α-methylbenzyl)salicyclic acid and 2-hydroxy-1-benzyl-3-naphthoic acid, phenolic compounds, e.g., 4,4'-isopropylidenediphenol, 4,4'-isopropylidenebis(2-chlorophenyl), 4,4'-isopropylidenebis(2,6-dibromophenol), 4,4'-isopropylidenebis(2,6-dichlorophenol), 4,4'-isopropylidenebis(2-methylphenol), 4,4'-isopropylidenebis(2,6-dimethyl)phenol, 4,4'-isopropylidenebis(2-tert-butylphenol), 4,4'-sec-butylidenebisphenol, 4,4'-cyclohexylidenebisphenol, 4,4'-cyclohexylidenebis(2-methylphenol), 4-tert-butylphenol, 4-phenylphenol, 4-hydroxydiphenoxide, α-naphthol, β-naphthol, methyl-4-hydroxybenzoate, benzyl-4-hydroxybenzoate, 2,2'-thiobis(4,6-dichlorophenol), 4-tert-octylcatechol, 2,2'-methylenebis(4-chlorophenol), 2,2'-methylenebis(4-methyl-6-tert-butyphenol) and 2,2'-dihydroxydiphenyl, phenol resins, e.g., p-phenylphenol-formaldehyde resin and p-butylphenol-acetylene resin; salts of the above organic acceptors with polyvalent metals such as zinc, magensium, aluminium, calcium, titanium, manganese, tin and nickel; and inorganic acid such as hydrogen halide, e.g., hydrogen chloride, hydrogen bromide and hydrogen iodide, boric acid, silicic acid, phosphoric acid, sulfuric acid, nitric acid, perchloric acid and halides of aluminium, zinc, nickel, tin, titanium and boron.

In cases of electron beam record materials or photosensitive record materials, compounds which can produce by electron beam or light radiation hydrogen halides, such as hydrogen chloride, hydrogen bromide and hydrogen iodide, carboxylic acids, sulfonic acids or phenols are preferably used as acceptor materials. Among those compounds, there are included organic halogen compounds, such as carbon tetrabromide, α,α,α-tribromoacetophenone, hexachloroethane, iodoform, 2-tribromomethylpyridine, trichloromethyl sulfonylbenzene, o-quinonediazido compounds, phenol esters of carboxylic acid or sulfonic acid which can cause Fries rearrangement.

Some embodiments of the utilization of the phthalide derivatives according to the invention for various kinds of record materials described hereinbelow:

The phthalide derivatives can be utilized for various kinds of pressure sensitive record materials, e.g.,

those disclosed in US Patent Specifications Nos. 2,505,470, 2,505,471 2,505,489, 2,548,366, 2,712,507, 2,730,456, 2,730,457, 3,418,250, 3,924,027 and 4,010,038.

A typical method for the production of a pressure sensitive record material utilizing the phthalide derivatives according to the invention is as follows:

At least one of the phthalide derivatives according to the invention is dissolved in a solvent to form a solution which may include synthetic oil such as alkylated naphthalene, alkylated diphenyl, alkylated diphenylmethane and alkylated terphenyl, vegetable oil such as cotton seed oil and caster oil, animal oil and mineral oil or mixtures of the foregoing. The solution may additionally include basic colourless chromogenic material such as triarylmethane lactones, spiropyrans, fluorans diphenylmethanes and Leucomethylene Blue. The solution of the phthalide derivative may be dispersed in a binder to form a coating composition. The solution may be enclosed in microcapsules through the utilization of the coacervation technique, the interfacial polymerization technique, the in-situ polymerization technique or any other method for making oil droplet-containing microcapsules and the microcapsules thus prepared are dispersed in a binder to form a coating composition.

Any one of the compositions thus prepared is applied to a base sheet such as a paper sheet, plastic sheet, resin coated paper sheet, to obtain a pressure sensitive record material. In case where the pressure sensitive copying system consists of a top sheet, a bottom sheet and, if necessary, at least one middle sheet, the pressure sensitive record material according to the invention is used as the top sheet and the middle sheet. The pressure sensitive material according to the invention also be utilized in the "self contained" system in which both the colourless chromogenic material and the acceptor are disposed on one surface of the same sheet. The pressure sensitive record material utilizing the phthalide derivative according to the invention can produce clear colour images having a good light resistance and showing a good absorption for infrared rays which enables a certain reading by an optical reading machine.

The phthalide derivatives according to the invention are also useful for production of various kinds of heat sensitive record materials, e.g., as disclosed in Japanese Patent Publications Nos. 3,680 of 1969, 27,880 of 1969, 14,039 of 1970, 43,830 of 1973, 69 of 1974, 70 of 1974 and 20,142 of 1977. Most typically, heat sensitive record materials may be produced by coating a coating composition including a binder, fine particles of the phthalide derivative according to the invention and the acceptor on a base sheet such as paper sheet, plastic film, synthetic paper sheet, woven fabric sheet or mold.

The amount of the acceptor in the recording layer may be within the range of 1 to 50 parts by weight, preferably within the range of 4 to 10 parts by weight, per one part by weight of the phthalide derivative used. The coating composition may include inorganic metal compounds such as oxides, hydroxides and carbonates of polyvalent metals and/or inorganic pigments in an amount of 0.1 to 5 parts by weight, preferably, 0.2 to 2 parts by weight, per one part by weight of the amount of the acceptor. The recording layer may also include dispersing agents, ultra-violet ray absorbing agents, heat fusible materials, antifoaming agent, fluorescent dye, colouring dyes and other adding materials. The phthalide derivative and the acceptor may be applied to a base sheet either in the form of a single coating composition or in the form of two separate coating compositions which may be applied one by one. Application of the phthalide derivative and acceptor to a base sheet may also be carried out by impregnation or by sizing. The amount of the coating composition including the phthalide derivative and the acceptor may preferably be within the range of 2 to 12 g/cm$^2$. Among the useful binder materials there may be included starches, celluloses, proteins, gum arabic, polyvinyl alcohol, salts of sytrenemaleic anhydride copolymer, styrene-butadiene copolymer emulsions, salts of vinyl acetate-maleic anhyride copolymer and salts of polyacrylic acid.

The electrothermal record materials may be produced according to any known methods such as those disclosed in Japanese Laid-Open Patent Publications No. 11,334 of 1974 and 48,930 of 1975. Usually, the record material of this type may be produced, either by coating on a base sheet such as a paper sheet a coating composition consisting of a dispersion of an electro-conductive material a basic dye material essentially comprising the phthalide derivative according to the invention, an acceptor and a binder, or by coating an electro-conductive material on a basic sheet to form an electro-conductive layer thereon and further coating on the electro-conductive layer another coating composition consisting of a dispersion of the phthalide derivative according to the invention, an acceptor and a binder. In case where each of the phthalide derivative and the acceptor used is not fusible within the temperature range of 70 to 120°C, an appropriate heat fusible material may be added for controlling the heat sensitivity.

The photosensitive record materials in which the phthalide derivatives according to the invention are utilized may be produced in a similar manner to any of those disclosed in Japanese Patent Publications Nos. 24,188 of 1963, 10,550 of 1970, 13,258 of 1970, 204 of 1974, 6,212 of 1974 and 28,449 of 1974 and Japanese Laid-Open Patent Publication Nos. 31,615 of 1972, 32,532 of 1973, 9,227 of 1974, 135,617 of 1974, 80,120 of 1975, 87,317 of 1975 and 126,228 of 1975.

The invention is also applicable to other recording systems, such as, the ultrasonic record material, e.g., a disclosed in French Patent Specification No. 2,120,992, the electron beam recording system, e.g., as disclosed in Belgian Patent No. 7,959,986, the electrostatic record material, e.g., as disclosed in Japanese Patent Publication No. 3,932 of 1974, the photosensitive printing material, e.g., as disclosed in Japanese Laid-Open Patent Publication No. 12,104 of 1973, the seal stamping material, e.g., as disclosed in Japanese Patent Publication No. 10,766 of 1972, type ribbons as disclosed in Japanese Laid-Open Patent Publication No. 3,713 of 1974, ball-point pen ink as disclosed in Japanese Laid-Open Patent Publication No. 83,924 of

1973 and crayon as disclosed in US Patent Specification No. 3,769,045, by merely using the phthalide derivatives instead of the conventional basic colourless chromogenic materials.

The following examples serve to illustrate the invention in more detail. Unless otherwise indicated, parts and % signify parts by weight and % by weight, respectively.

Example 1

30 g of 2-p-methoxybenzoyl-5-dimethylaminobenzoic acid and 27 g of 1,1-bis(p-dimethylaminophenyl)ethylene were added to 60 g of acetic anhydride and the mixture was heated at 50°—55°C for 30 minutes with stirring. After the termination of reaction, sodium hydroxide was added to the mixture and to decompose acetic anhydride. The resultant precipitate was separated by filtration and then dissolved in hydrochloric acid. The solution was filtered and neutralized with an aqueous solution of sodium hydroxide to obtain a solid. The solid was separated by filtration, dried and recrystallized from methanol to obtain 50.3 g of 3 - (p - methoxyphenyl) - 3 - [1,1 - bis(p - dimethylaminophenyl)ethylene - 2 - yl] - 6 - dimethylaminophthalide in the form of light blue crystals having a melting point of 103°—105°C. The phthalide derivative became blue black upon contact with silica gel.

Example 2

Example 1 was repeated except that 33 g of 2-(3,4-dimethoxy)benzoyl-5-dimethylaminobenzoic acid was used instead of 30 g of 2-p-methoxybenzoyl-5-dimethylaminobenzoic acid to obtain 52.0 g of 3 - (3,4 - dimethoxyphenoxy) - 3 - {1,1 - bis(p - dimethylaminophenyl)ethylene - 2 - yl} - 6 - dimethylaminophthalide in the form of light blue crystals having a melting point of 104°—107°C. The phthalide derivative became blue black upon contact with silica gel.

Example 3

Example 1 was repeated except that 28 g of 2-p-methylbenzoyl-5-dimethylaminobenzoic acid was used instead of 30 g of 2-p-methoxybenzoyl-5-dimethylaminobenzoic acid to obtain 46.7 g of 3 - (p - methylphenyl) - 3 - {1,1 - bis(p - dimethylaminophenyl)ethylene - 2 - yl} - 6 - dimethylaminophthalide in the form of light blue crystals having a melting point of 113—116°C. The phthalide derivative became green blue upon contact with silica gel.

Example 4

Example 1 was repeated except that 31 g of 1-(4-dimethylaminophenyl)-1-(4-diethylamino-2-methylphenyl)ethylene was used instead of 27 g of 1,1-bis(p-dimethylaminophenyl)ethylene to obtain 32.8 g of 3 - (p - methoxyphenyl) - 3 - {1 - (4 - dimethylaminophenyl) - 1 - (4 - diethylamino - 2 - methylphenyl)ethylene - 2 - yl} - 6 - dimethylaminophthalide in the form of light blue crystals having a melting point of 108°—111°C. The phthalide derivative became blue black upon contact with silica gel.

Examples 5 to 18

Example 1 was repeated except that benzophenon derivatives shown in Table 1 were used instead of 2-p-methoxybenozyl-5-dimethylaminobenzoic acid and ethylene derivatives shown in Table 1 were used instead of 1,1-bis(p-dimethylaminophenyl)ethylene to obtain phthalide derivatives shown in Table 1. The colors formed upon contact with silica gel are shown in Table 1.

| Example | Benzophenone derivatives | Ethylene derivatives | Phthalide derivatives | Colour |
|---|---|---|---|---|
| 5 | 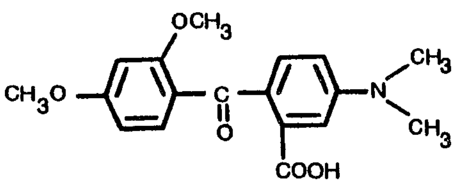 | 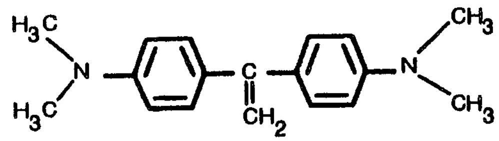 | 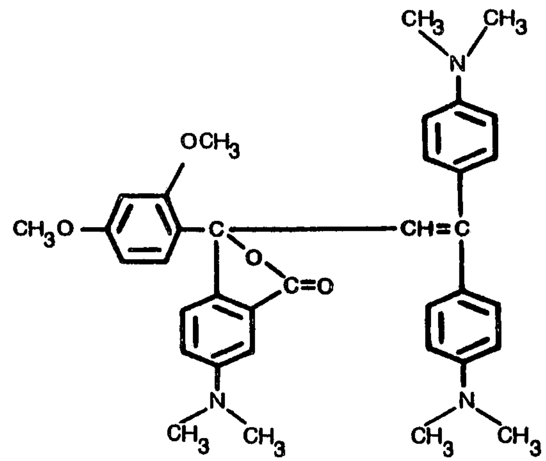 | black blue |
| 6 | 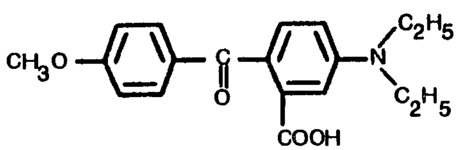 | 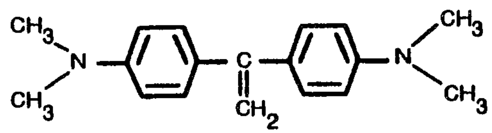 | 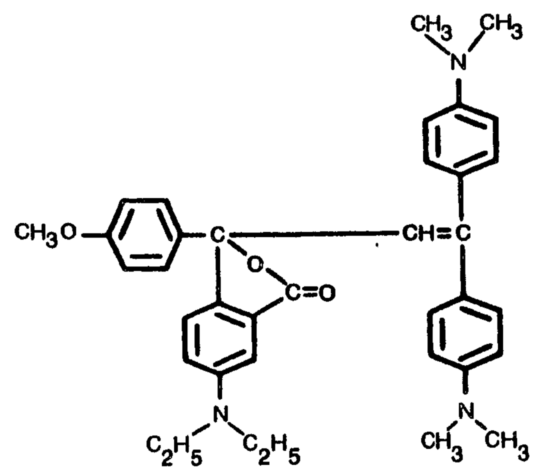 | black blue |

| Example | Benzophenone derivatives | Ethylene derivatives | Phthalide derivatives | Colour |
|---|---|---|---|---|
| 7 | 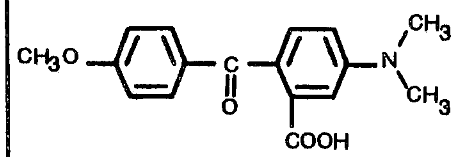 | 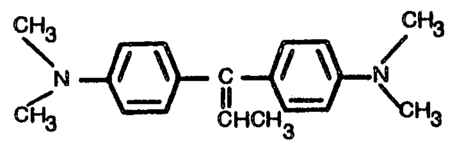 | 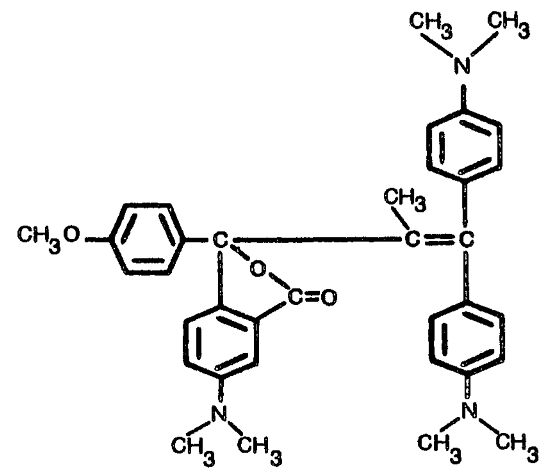 | black blue |
| 8 | 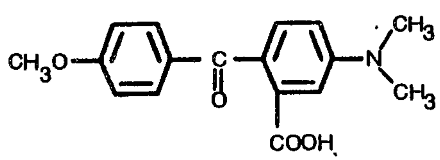 | 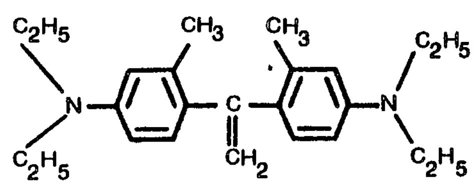 | 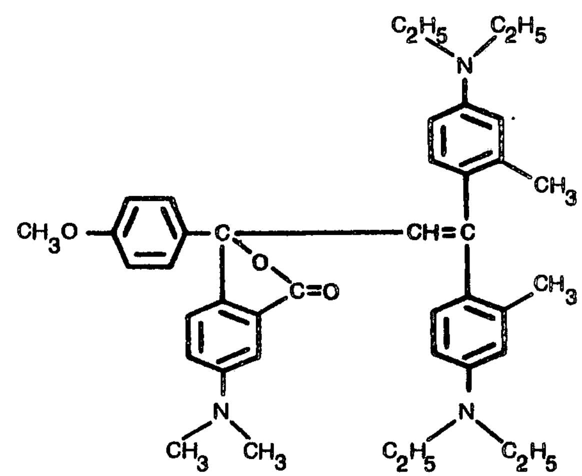 | black blue |

| Example | Benzophenone derivatives | Ethylene derivatives | Phthalide derivatives | Colour |
|---|---|---|---|---|
| 9 | 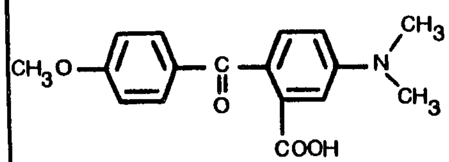 | 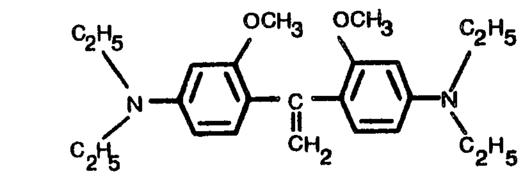 | 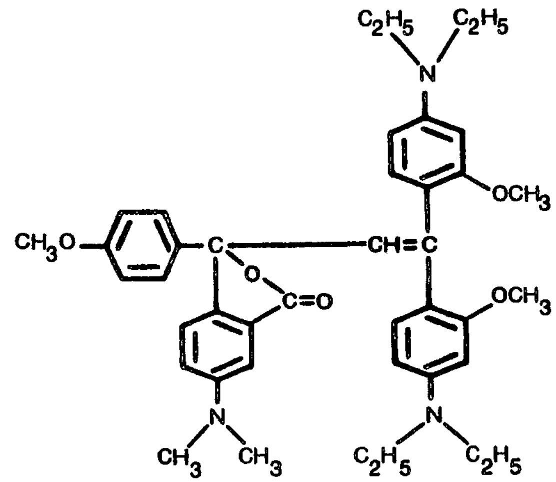 | black blue |
| 10 | 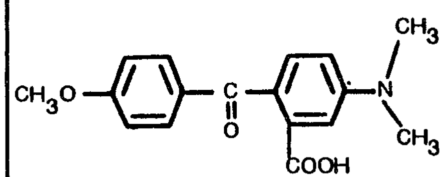 | 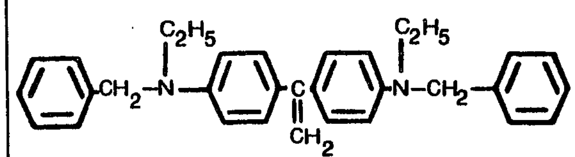 | 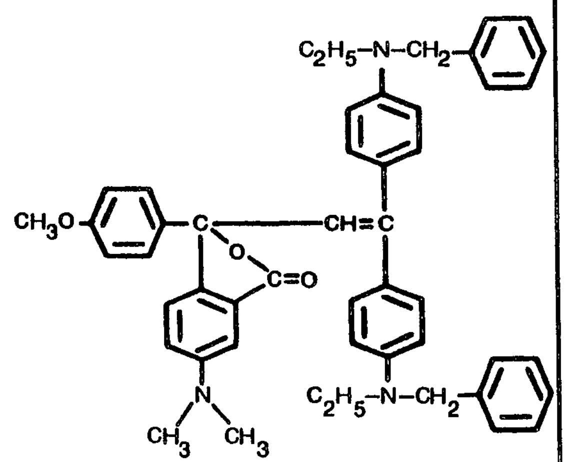 | black blue |

| Example | Benzophenone derivatives | Ethylene derivatives | Phthalide derivatives | Colour |
|---|---|---|---|---|
| 11 | 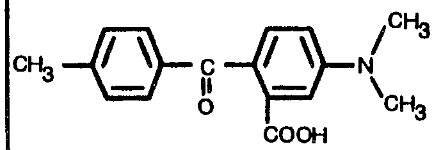 | 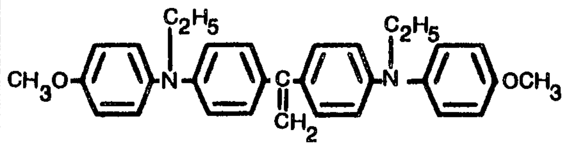 | 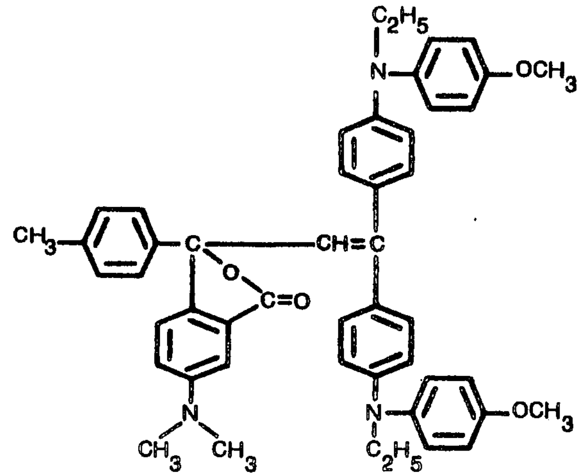 | blue |
| 12 | 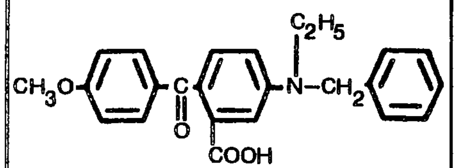 | 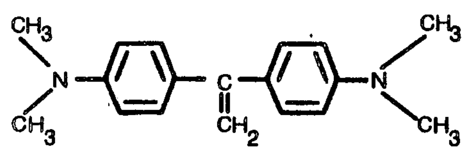 | 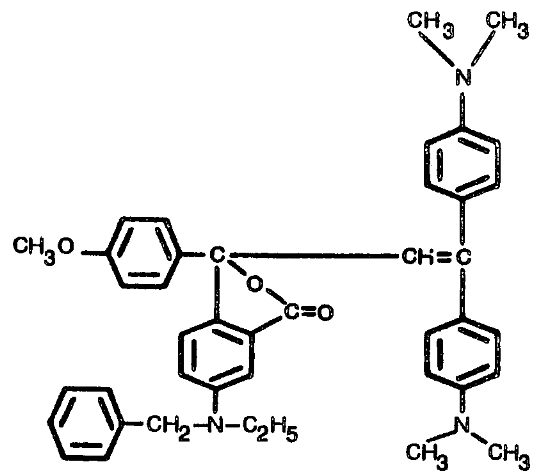 | black blue |

| Example | Benzophenone derivatives | Ethylene derivatives | Phthalide derivatives | Colour |
|---|---|---|---|---|
| 13 | 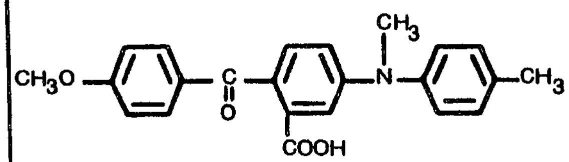 | 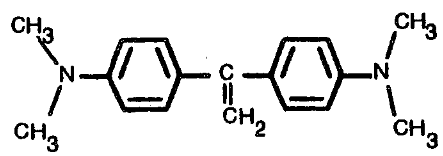 | 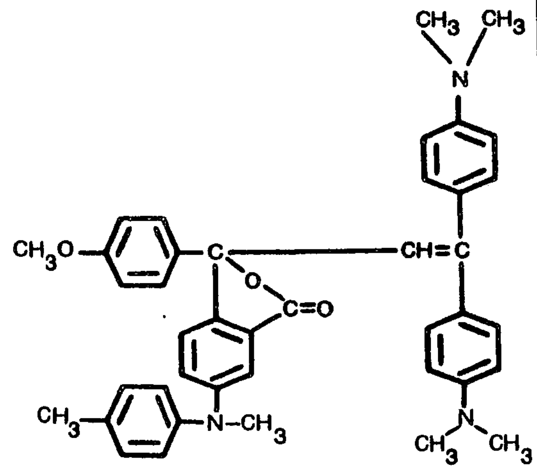 | black blue |
| 14 | 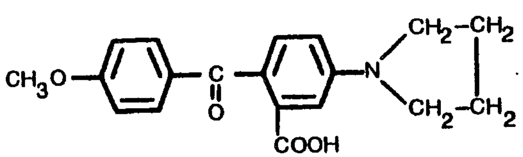 | 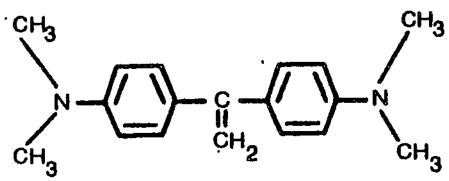 | 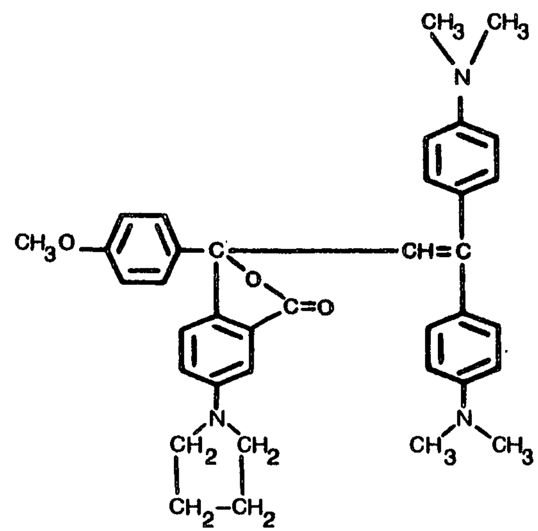 | black blue |

| Example | Benzophenone derivatives | Ethylene derivatives | Phthalide derivatives | Colour |
|---|---|---|---|---|
| 15 | 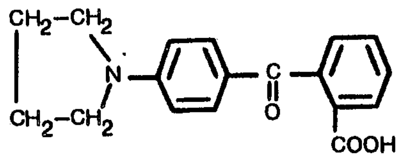 | 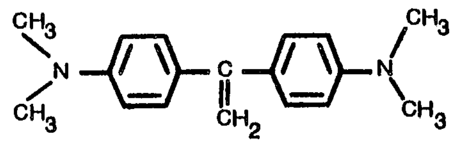 | 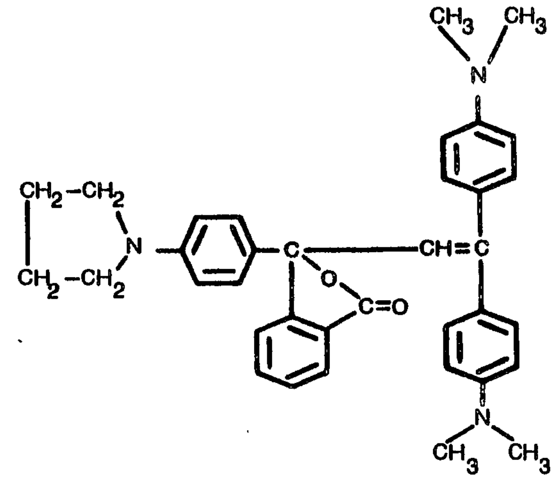 | blue green |
| 16 | 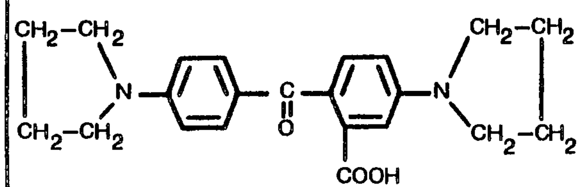 | 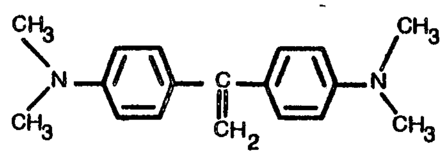 | 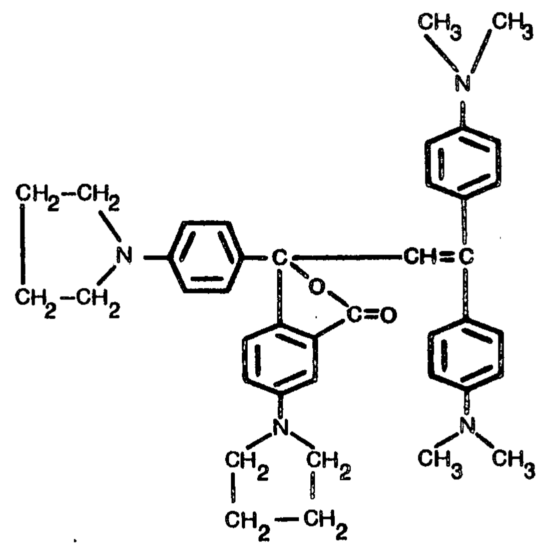 | blue violet |

| Example | Benzophenone derivatives | Ethylene derivatives | Phthalide derivatives | Colour |
|---|---|---|---|---|
| 17 | 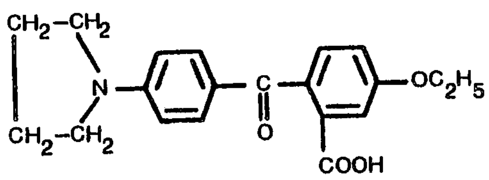 | 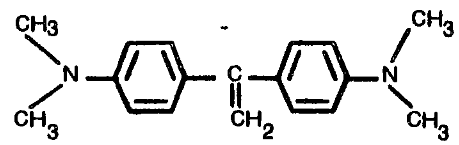 | 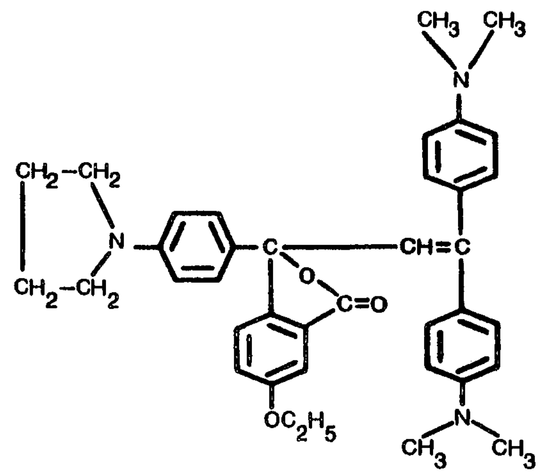 | blue green |
| 18 | 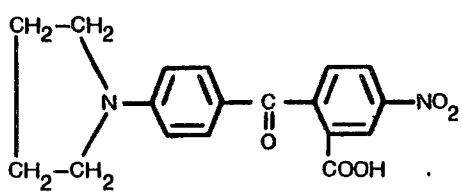 | 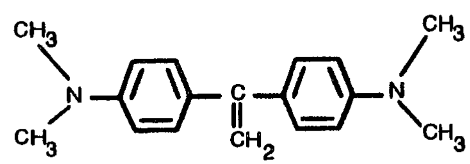 | 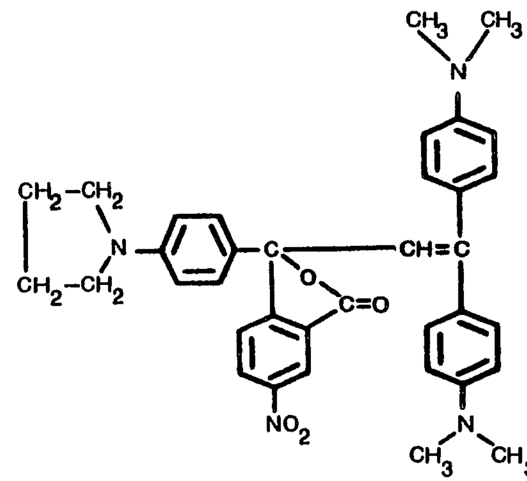 | blue green |

Example 19

A heat-sensitive record material was prepared by the following method with the use of 3-(p-methoxyphenyl)-3-{1,1-bis(p-dimethylaminophenyl)ethylene-2-yl}-6-dimethylaminophthalide obtained in Example 1.

(1) Preparation of A liquid:

The following composition was passed through a sand mill

| | |
|---|---|
| phthalide derivative obtained in Example 1 | 5 parts |
| stearic acid amide | 1 part |
| 2% aqueous solution of hydroxyethylcellulose | 25 parts |

Pulverization was continued until an average particle size of 2 microns.

(2) Preparation of B liquid:

The following composition was passed through a sand mill

| | |
|---|---|
| 4,4'-isopropylidenediphenol | 50 parts |
| stearic acid amide | 10 parts |
| 2% aqueous solution of hydroxyethylcellulose | 250 parts |

Pulverization was continued until an average particle size of 2 microns.

(3) Making a heat-sensitive record material:

The following composition was mixed to prepare a coating composition.

| | |
|---|---|
| A liquid | 62 parts |
| B liquid | 31 parts |
| ultrafinely divided particles of silicic anhydride ("Syloid 244" manufactured by Fuji-Davidson Chemical) | 25 parts |
| 20% aqueous solution of a salt of stryrene-maleic anhydride copolymer | 175 parts |
| Zinc stearate | 5 parts |
| water | 100 parts |

The coating composition was coated on a base sheet of 50 $g/m^2$ in the weight of an amount of 6 $g/m^2$ on dry basis to obtain a heat-sensitive record material.

The heat-sensitive record material was pressed with a pressure of 4 $kg/cm^2$ for 5 seconds on a plate heated at 125°C to develop black blue images. The colour images were superior in light resistance. The colour change and discoloration when exposed to sunlight were not substantially appreciated. The light absorption spectrum of the colour images had a strong maximum absorption at 510 nm and a broad strong absorption at 580—800 nm.

Example 20

A pressure-sensitive record material was prepared by the following method with the use of 3-p-pyrrolidinophenyl-3-{1,1-bis(p-dimethylaminophenyl)ethylene-2-yl}-6-pyrrolidinophthalide obtained in Example 16.

3 parts of the above phthalide derivative was dissolved in 100 parts of isopropylated naphthalene. The resultant solution was dispersed in 350 parts of warm water (50°C) containing 25 parts of pigskin-gelatin having an isoelectric point of 8 and 25 parts of gum arabic dissolved in it to obtain an emulsion. 1000 parts of warm water was added to the emulsion. The mixture was adjusted to pH 4 with acetic acid and cooled at 10°C. 10 parts of 25% aqueous solution of glutaraldehyde was added to it to solidify capsules. The capsule-containing coating composition was coated on one surface of a base sheet of 45 $g/m^2$ in the weight of 5 $g/m^2$ on dry basis and an acceptor coating composition comprising 20 parts of zinc 3,5-bis(α-methylbenzyl)-salicylate, 80 parts of kaolin and 30 parts of styrene-butadiene copolymer emulsion (solid content: 50%)

dispersed in 200 parts of water was coated on another surface of the base sheet in the weight of 5 $g/m^2$ on dry basis to obtain a pressure-sensitive record material (middle sheet).

Several of the pressure-sensitive record material were piled in the manner as the capsule coated layer was closed to the acceptor coated layer, pressed with driving a pen to obtain blue color images on the acceptor coated surface. The color images were stable to water and alcohol and when exposed to sunlight the color change and discoloration were not appreciated. The light absorption spectrum had strong maximum absorptions at 595 nm, 670 nm and 760 nm.

Example 21

Example 19 was repeated except that 3 parts of 3-p-pyrrolidinophenyl-3-{1,1-bis(p-dimethylaminophenyl)ethylene-2-yl}-phthalide obtained in Example 15 was used instead of 3 parts of 3-p-pyrrolidinophenyl-3-{1,1-bis(p-dimethylaminophenyl)ethyele-2-yl}-6-pyrrolidinophthalide to obtain a pressure-sensitive record material and to develop colour images. The colour images were superior in light resistance and the light absorption spectrum of the colour images had strong maximum absorptions at 650 nm and 750 nm and a weak maximum absorption at 460 nm.

Example 22

An electrothermal record material was prepared by the following method with the use of the phthalide derivative obtained in Example 2.

200 parts of cuprous iodide and 5 parts of 10% aqueous solution of sodium sulfite were added to 200 parts of 1% aqueous solution of polyvinyl alcohol. The mixture was passed through a sand mill. Pulverization was continued until an average particle size of 2 microns. To the pulverized mixture 8 parts of polyacrylate emulsion and 20 parts of titanium dioxide were added and thoroughly dispersed. The dispersion was coated on a base sheet of 50 $g/m^2$ in the weight of 7 $g/m^2$ on dry basis. Further, there was coated on the coating layer in the weight of 5 $g/m^2$ on dry basis a heat-sensitive coating composition prepared by the same manner as in Example 19 except that 3-(3,4-dimethoxyphenyl)-3-{1,1-bis(p-dimethylaminophenyl)ethylene-2-yl}-6-dimethylaminophthalide obtained in Example 2 was used instead of 3-(p-methoxyphenyl)-3-{1,1-bis(p-dimethylaminophenyl)ethylene-2-yl}-6-dimethylaminophthalide to obtain an electrothermal record material.

Images were recorded on the record material with the use of a cylindrical scanning recording machine at a scanning speed of 630 mm/sec with a needle pressure of 10 g. The recorded images were dark blue and superior in light resistance. The light adsorption spectrum of them had a strong maximum absorption at 500 nm and a strong broad adsorption at 600—800 nm.

Example 23

A photosensitive record material was prepared by the following method with the use of the phthalide derivative obtained in Example 5.

6 g of 3-(2,4-dimethoxy)-3-{1,1-bis(p-dimethylaminophenyl)ethylene-2-yl}-6-dimethylaminophthalide obtained in Example 5 was dissolved in 40 ml of chloroform. 40 ml of 10% benzene solution of polystyrene and 5 g of carbon tetrabromide were added to the solution and the mixture was thoroughly stirred to prepare a coating composition. The coating composition was coated on polyethylene laminated paper having polyethylene at the both surfaces in the weight of 5 $g/m^2$ on dry basis in a dark place. The coated paper was irradiated with a light of eight ultraviolet lamps of 20W from a distance of 5 cm for 10 minutes to develop blue colour images. The colour images were then fixed by rinsing with a solution of acetone/n-hexane(1/5). The resultant images were stable when exposed to sunlight and the light absorption spectrum had a strong maximum absorption at 530 nm and a strong broad absorption at 600—800 nm.

Example 24

An ultrasonic vibrator of needle type having a radius of 0.2 mm was slightly contacted on a surface of the heat-sensitive record material obtained in Example 19 and the record material was moved at a speed of 20 cm/sec under an ultrasonic vibration of 19 KHz 20W to obtain blue recorded images superior in light resistance.

## Claims

1. A phthalide derivative having the general formula (I)

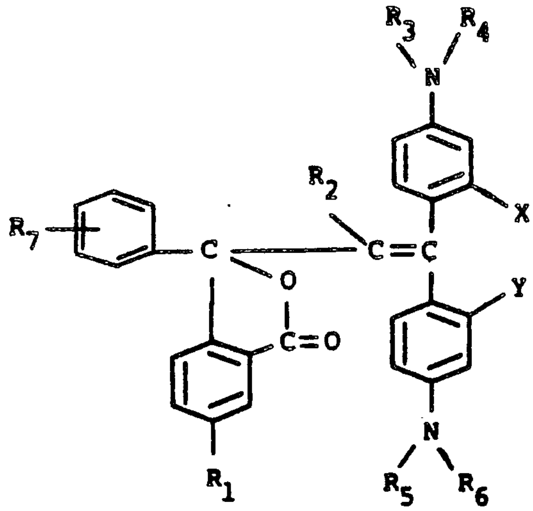

I

wherein $R_1$ is hydrogen, $C_{1-4}$ alkoxy, nitro, pyrrolidino or a substituted amino group of the formula

$-N(CH_3)_2$ , $-N(C_2H_5)_2$ , 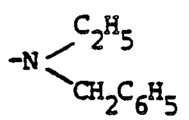 or 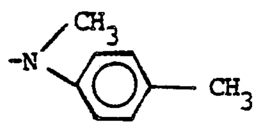 ;

$R_2$ is hydrogen or methyl;

each of $R_3$, $R_4$, $R_5$ and $R_6$ is $C_{1-4}$ alkyl, benzyl, halogen-substituted benzyl, phenyl, or $C_{1-2}$ alkyl or $C_{1-2}$ alkoxy-substituted phenyl, or $R_3$ and $R_4$ together with the adjacent nitrogen atom and/or $R_5$ and $R_6$ together with the adjacent nitrogen atom form pyrrolidino, piperidino, hexamethyleneimino or morpholino;

$R^7$ is one or two substituents selected from $C_{1-4}$ alkyl, $C_{1-4}$ alkoxy and pyrrolidino;

each of X and Y is hydrogen, $CH_3$ or $OCH_3$.

2. A phthalide derivative according to claim 1, of formula (I) where $R_1$ is a substituted amino group represented by the general formula

$-N(CH_3)_2$ , $-N(C_2H_5)_2$ , 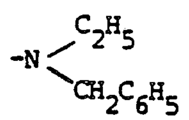 or 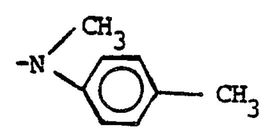 ; and

wherein $R_3$ and $R_5$ are the same, $R_4$ and $R_6$ are the same and X and Y are the same.

3. A phthalide derivative according to claim 1, in which said phthalide derivative has the general formula (II)

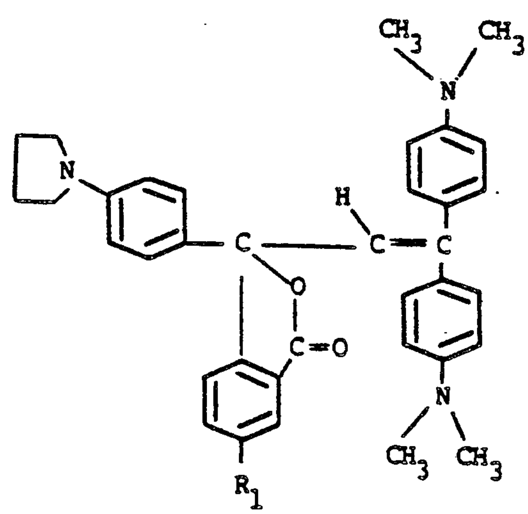

II

where $R_1$ is hydrogen, pyrrolidino, ethoxy or nitro.

4. A phthalide derivative according to claim 1, of the formula (III)

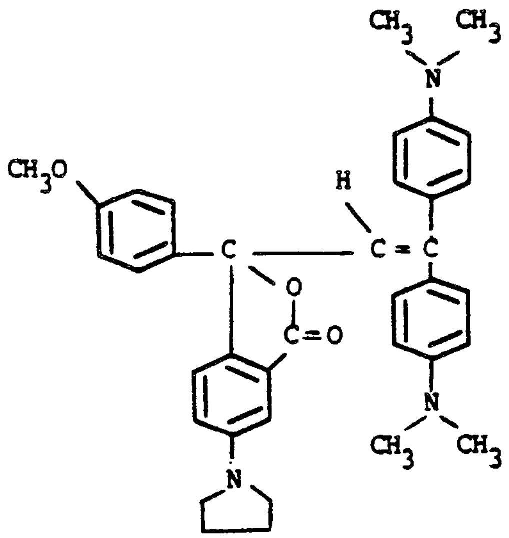

III

5. A process for preparing a phthalide derivative as defined in any of claims 1 to 4, which comprises reacting a benzophenone derivative having the general formula (IV)

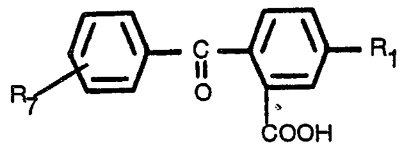

IV

with an ethylene derivative having the general formula (V)

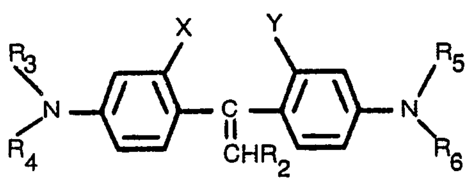

V

wherein $R_1$, $R_2$, $R_3$, $R_4$, $R_5$, $R_6$, $R_7$, X and Y are defined in any of claims 1 to 4.

6. A recording system which utilizes the colour forming reaction between a colourless chromogenic material and an electron accepting acidic reactant material characterized in that said colourless chromogenic material comprises a phthalide derivative as defined in any of claims 1 to 4.

7. A recording system according to Claim 6, in which said recording system is a pressure-sensitive recording system.

8. A recording system according to Claim 6, in which said recording system is a heat-sensitive recording system.

**Patentansprüche**

1. Ein Phthalsäure-Derivat der allgemeinen Formel (I)

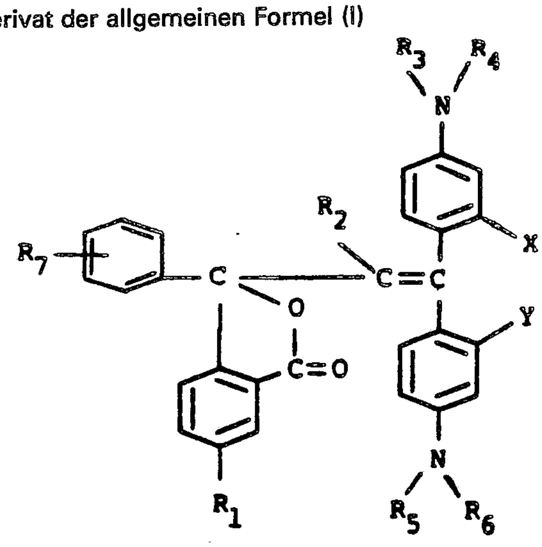

I

worin

$R_1$ Wasserstoff, eine $C_{1-4}$-Alkoxy-, Nitro-, Pyrrolidino-Gruppe oder eine substituierte Aminogruppe der Formel

$-N(CH_3)_2$ , $-N(C_2H_5)_2$ , 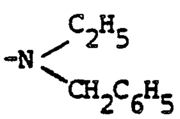 oder 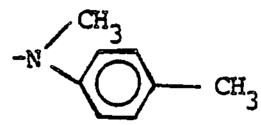 ;

worin

$R_2$ Wasserstoff oder Methyl ist;

$R_3$, $R_4$, $R_5$ und $R_6$ jeweils eine $C_{1-4}$-Alkyl-, Benzyl-, halogensubstituierte Benzyl-, Phenyl- oder $C_{1-2}$-Alkyl- oder $C_{1-2}$ alkoxy-substituierte Phenyl-Gruppe bedeuten, oder $R_3$ und $R_4$ zusammen mit dem benachbarten Stickstoffatom und/oder $R_5$ und $R_6$ zusammen mit dem benachbarten Stickstoffatom eine Pyrrolidino-, Piperidino-, Hexamethylenimino- oder Morpholino-Gruppe bilden;

$R_7$ ein oder zwei Substituenten jeweils mit der Bedeutung einer $C_{1-4}$-Alkyl-, $C_{1-4}$-Alkoxy- oder Pyrrolidino-Gruppe bedeutet; und X und Y jeweils Wasserstoff, $CH_3$ oder $OCH_3$ sind.

2. Ein Phthalsäure-Derivat nach Anspruch 1 der Formel (I), worin $R_1$ eine substituierte Aminogruppe der allgemeinen Formel ist

$-N(CH_3)_2$ , $-N(C_2H_5)_2$ , 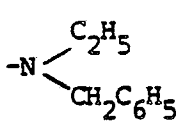 oder 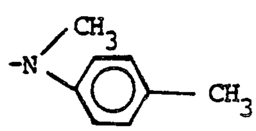 ,

worin $R_3$ und $R_5$ sowie $R_4$ und $R_6$ sowie X und Y jeweils die gleiche Bedeutung haben.

3. Ein Phthalsäure-Derivat nach Anspruch 1 der allgemeinen Formel (II)

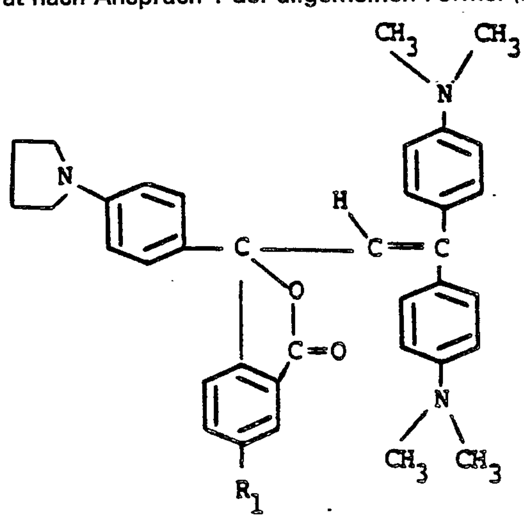

II

worin $R_1$ Wasserstoff, eine Pyrrolidino-, Ethoxy- oder Nitro-Gruppe ist.

4. Ein Phthalsäure-Derivat nach Anspruch 1 der allgemeinen Formel (III)

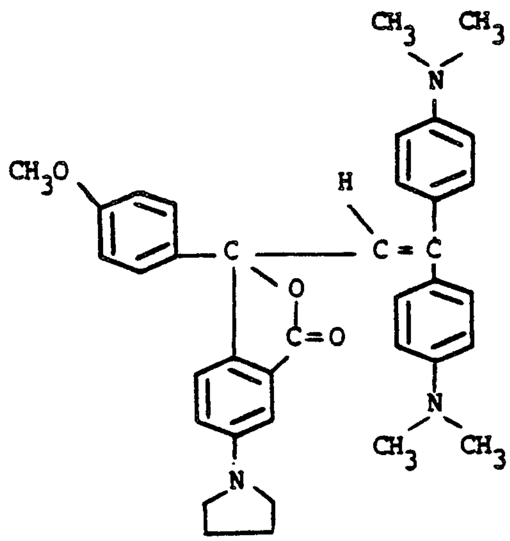

III

5. Verfahren zur Herstellung eines Phthalsäure-Derivates nach einem der Ansprüche 1 bis 4, wobei ein Benzophenon-Derivat der allgemeinen Formel (IV)

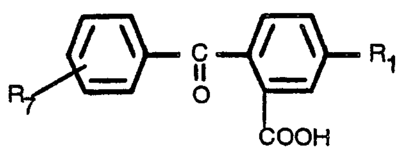

IV

mit einem Ethylene-Derivat der allgemeinen Formel (V)

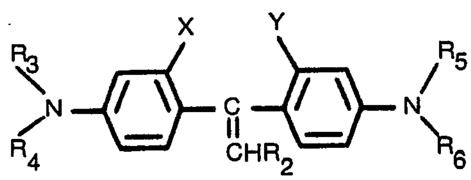

V

umgesetzt wird,

worin $R_1$, $R_2$, $R_3$, $R_4$, $R_5$, $R_6$, $R_7$, X und Y die in den Ansprüchen 1 bis 4 angegeben Definition besitzen.

6. Ein Aufzeichnungssystem, das die farbbildende Reaktion zwischen einem farblosen chromogenen Material und einem Elektronen aufnehmenden saueren Reaktanten-Material nutzt, dadurch gekennzeichnet, daß das farblose chromogene Material ein Phthalsäure-Derivat nach einem der Ansprüche 1 bis 4 umfaßt.

7. Ein Aufzeichnungssystem nach Anspruch 6, wobei das Aufzeichnungssystem ein drucksensibles Aufzeichnungssystem ist.

8. Ein Aufzeichnungssystem nach Anspruch 6, wobei das Aufzeichnungssystem ein hitzesensibles Aufzeichnungssystem ist.

**Revendications**

1. Un dérivé phtalide de formule générale (I)

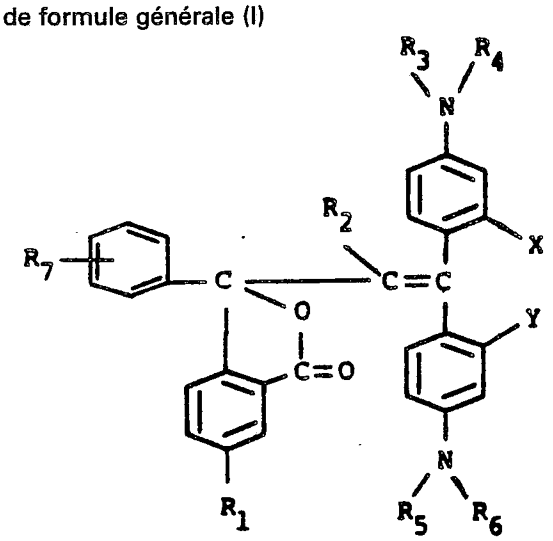

I

dans laquelle

$R_1$ est hydrogène, un groupe alkoxy en $C_1$—$C_4$, nitro, pyrrolidino ou un groupe amino substitué de formule

$-N(CH_3)_2$ , $-N(C_2H_5)_2$ , 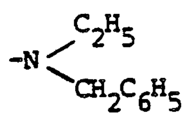 ou 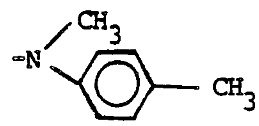

$R_2$ est un hydrogène ou un groupe méthyle, chaque substituant $R_3$, $R_4$, $R_5$ et $R_6$ est un groupe alkyle en $C_1$—$C_4$, benzyle, phényle, ou $C_1$—$C_2$ alkyle ou $C_1$—$C_2$ alkoxy- phényle, ou $R_3$ et $R_4$ ensemble avec l'atome d'azote adjacent et/ou $R_5$ et $R_6$ ensemble et l'atome d'azote adjacent forment un groupe pyrrolidino, pipéridino, hexaméthyleneimino ou morpholino,

$R_7$ représente un ou deux substituants choisis parmi un groupe alkyl en $C_1$—$C_4$, un groupe alkoxy en $C_1$—$C_4$ ou un groupe pyrrolidino;

X et Y sont chacun un hydrogène, un groupe —$CH_3$ ou un groupe —$OCH_3$.

2. Un dérivé phtalide selon la revendication 1 de formule (I) dans laquelle $R_1$ est un groupe amino substitué represénté par la formule générale

$-N(CH_3)_2$ , $-N(C_2H_5)_2$ , 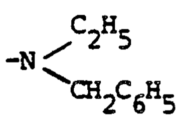 ou 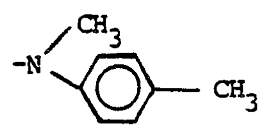 ;

et dans laquelle $R_3$ et $R_5$, $R_4$ et $R_6$ sont identiques et X et Y sont identiques.

3. Un dérivé phtalide selon la revendication 1 dans laquelle ledit dérivé phtalide est de formule générale (II)

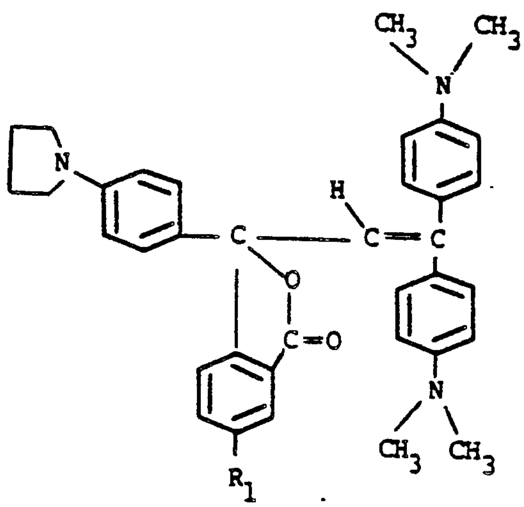

II

dans laquelle $R_1$ est hydrogène, pyrrolidino, éthoxy ou nitro.

4. Un dérivé phtalide selon la revendication 1 de formule (III)

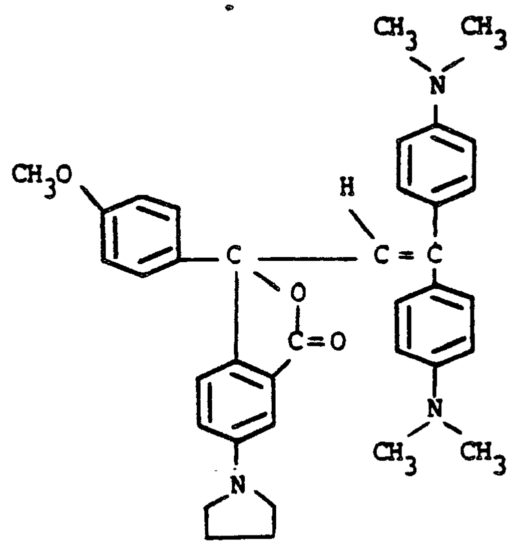

III

5. Procédé de préparation d'un dérivé phtalide comme défini dans n'importe laquelle des revendications 1 à 4, comprenant une étape de réaction d'un dérivé de la benzophénone de formule générale (IV)

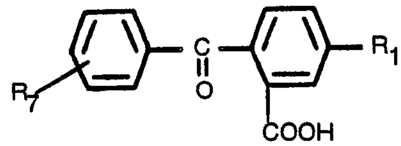

IV

avec un dérivé éthylénique de formule générale (V)

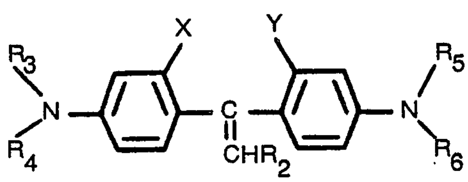

V

dans lesquelles $R_1$, $R_2$, $R_3$, $R_4$, $R_5$, $R_6$, $R_7$, X et Y sont comme définis dans n'importe laquelle des revendications 1 à 4.

6. Un système d'enregistrement utilisant la réaction de formation de couleur entre un produit chromogène incolore et un réactif acide accepteur d'electrons caractérisé en ce que ledit produit chromogène comprend un dérivé phtalide tel que défini dans n'importe laquelle des revendications 1 à 4.

7. Un système d'enregistrement selon la revendication 6, dans laquelle ledit système d'enregistrement est un système d'enregistrement basé sur la sensibilité à la pression.

8. Un système d'enregistrement selon la revendication 6, dans lequel le système d'enregistrement est un système d'enregistrement thermosensible.